# EUROPEAN PATENT SPECIFICATION

(11) **EP 2 046 727 B1**
(45) Date of publication and mention of the grant of the patent: **12.03.2014**
(21) Application number: 06780202.5
(22) Date of filing: 25.07.2006
(51) Int. Cl.: C07C 215/40, C07C 215/42, A61K 31/216, A61P 29/00, A61P 43/00

(54) **POSITIVELY CHARGED WATER-SOLUBLE PRODRUGS OF DICLOFENAC WITH VERY FAST SKIN PENETRATION RATE**
POSITIV GELADENE, WASSERLÖSLICHE PRODRUGS VON DICLOFENAC MIT SEHR SCHNELLER HAUTPENETRATIONSGESCHWINDIGKEIT
PROMÉDICAMENTS HYDROSOLUBLES À CHARGE POSITIVE DU DICLOFÉNAC PRÉSENTANT UNE VITESSE DE PÉNÉTRATION CUTANÉE TRÈS RAPIDE

(43) Date of publication of application: 15.04.2009
(73) Proprietor: Techfields Biochem Co. Ltd, Shanghai N/A 200444 (CN); Yu, Chongxi, Plainfield, Illinois 60585 (US)
(72) Inventor: YU, Chongxi, Plainfield IL 60585 (US); XU, Lina, N/A 200444 Shanghai (CN)
(74) Representative: Viering, Jentschura & Partner Patent- und Rechtsanwälte
(86) International application number: PCT/IB2006/052549
(87) International publication number: WO 2008/012602

(56) References cited:
- EP-A2- 0 289 262
- WO-A2-02/085297
- US-A- 4 640 911
- NEDIOGLU D ET AL: "Synthesis and in vitro anti-inflammatory activities of some new diaryl amine derivatives as prodrug of diclofenac" JOURNAL OF FACULTY OF PHARMACY OF GAZI UNIVERSITY, GAZI UNIVERSITY, ANKARA, TR, vol. 10, no. 1, 1 January 1993 (1993-01-01), pages 69-81, XP008110133 ISSN: 1015-9592
- J.-L. MAGNETTE ET AL: "Diclofenac systemic exposure is not increased when topical diclofenac is applied to ultraviolet-induced erythema" EUR. J. CLIN. PHARMACOL., vol. 60, 2004, pages 591-594, XP002590780
- L. APT ET AL: "A randomized clinical trial of the nonsteroidal eyedrop diclofenac after strabismus surgery" OPHTHALMOLOGY, vol. 105, no. 8, August 1998 (1998-08), pages 1448-1454, XP002590781
- TUNCA GUL ALTUNTAS ET AL.: 'A stud on the interation between p60c-src receptor tyrosine kinase and arylcarboxylic and arylacetic acid derivatives based on docking modes and in vitro activity' BIOL. PHARM. BULL. vol. 27, no. 1, 2004, pages 61 - 65, XP008102356
- CEVC G. AND BLUME G.: 'New, highly efficient formulation of diclofenac for the topical, transdermal administration in ultradeformable drug carriers, transersomes' BIOCHIM. BIOPHYS. ACTA vol. 1514, no. 2, 2001, pages 191 - 205, XP004319625
- PRIYABJA ARORA AND BISWAJIT MUKHERJEE: 'Design, development, physicochemical, and in vitro and in vivo evaluation of transdermal patches containing diclofenac diethylammonium salt' JOURNAL OF PHARMACEUTICAL SCIENCES vol. 91, no. 9, 2002, pages 2076 - 2089, XP008102312

## Description

### Technical Field

The present invention relates to the preparations of positively charged and water-soluble pro-drugs of 2[(2, 6-dichlorophenyl) amino] benzene acetic acid (diclofenac) and their medicinal use in treating any diclofenac-treatable conditions in humans or animals. More specifically, the present invention is to overcome the side effects that are associated with the use of diclofenac. These pro-drugs can be administered orally or transdermally.

### Background Art

Diclofenac is a member of the aryl- and heteroarylacetic acid group of nonsteroidal anti-inflammatory drugs. The synthesis of diclofenac was originally reported in 1969 (A. Sallman and R. Pfister, Ger. Patent No. 1,815,802). Diclofenac is available in 120 different countries and is perhaps the most widely used NSAIA in the world. Diclofenac possesses structural characteristics of both the arylalkanoic acid and the anthranilic acid classes of anti-inflammatory agents and displays anti-inflammatory, analgesic, and antipyretic properties. As an analgesic, it is 6 times more potent than indomethacin and 40 times more potent than aspirin. As an antipyretic, it is twice as potent as indomethacin and over 350 times as potent as aspirin. 'PDR Generics' (PDR Generics, 1996, second edition, Medical Economics, Montvale, New Jersey, pg 243) has listed many medical uses of diclofenac. Diclofenac is used for the relief of signs and symptoms of rheumatoid arthritis, osteoarthritis and ankylosing spondylitis. Its potassium salt is indicated for the treatment of dysmenorrhea. Diclofenac is used alone or as an adjunct in the treatment of biliary colic, fever, and episiotomy pain.. It is also used in treatment of gout, acute migraine headaches, and renal colic and in the treatment of postoperative inflammation in patients who have undergone cataract extraction.

Unfortunately, a number of side effects are associated with the use of diclofenac, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Pearson et al. U.S. Pat. No. 6,528,040, and Botknecht et al. U.S. Pat. No. 5,885,597) have attempted to develop a delivery system for transdermal application by formulation. Song, et al. developed a transdermal drug delivery system for anti-inflammatory analgesic agent comprising diclofenac diethylammonium salt (Song, et. al., US Patent No. 6,723,337). Donati, et al. developed a plaster for topical use containing heparin and diclofenac. (Donati, et al., US patent, NO. 6,592,891). Kawaji, et al. developed an oily patch for external use containing diclofenac sodium (Kawaji, et al. US Patent No. 6,262,121). Effing, et al. developed a device for the transdermal delivery of diclofenac (Effing, et al. US Patent No. 6,193,996).

Nedioglu et al. (Nedioglu, D. et al., Journal of faculty of pharmacy of Gazi University, 10, 1 (1993)) disclose hydrochloride salts of amine derivatives of diclofenac as well as their use as anti-inflammatory.

Patent document US 4 640 911 A discloses the hydrochloride salt of an aminobutylamide derivative of diclofenac.

Patent document EP 0 289 262 A2 discloses quaternary ammonium alkyl esters and thioesters of diclofenac as well as their synthesis.

It is very difficult, however, to deliver therapeutically effective plasma levels of diclofenac into the host by formulation. Susan Milosovich, et. al. designed and prepared testosteronyl-4- dimethylaminobutyrate.HCl (TSBH), which has a lipophilic portion and a tertiary amine groups that exists in the protonated form at physiological pH. They found that the prodrug (TSBH) diffuses through human skin ∼60 times faster than does the drug (TS) itself [Susan Milosovich, et al., J. Pharm. Sci., 82, 227(1993).

### Disclosure of Invention

### Technical Problem

Diclofenac has been used medicinally for more than 30 years and diclofenac is more potent than aspirin in anti-inflammatory and prostaglandin biosynthesis inhibition. Diclofenac is indicated for the relief of the signs and symptoms of rheumatoid arthritis and osteoarthritis, the relief of mild to moderate pain, the reduction of fever, and the treatment of dysmenorrhea. Diclofenac is available in 120 different countries and is perhaps the most widely used NSAIA in the world.

Unfortunately, a number of side effects are associated with the use of diclofenac, most notably GI disturbances such as dyspepsia, heartburn, vomiting, gastroduodenal bleeding, gastric ulcerations, and gastritis. Gastroduodenal bleeding induced by diclofenac is generally painless but can lead to fecal blood loss and may cause a persistent iron deficiency anemia.

### Technical Solution

This invention relates to the preparation of novel positively charged pro-drugs of diclofenac and their use medicinally. These pro-drugs have the formula (1) 'Structure 1'.

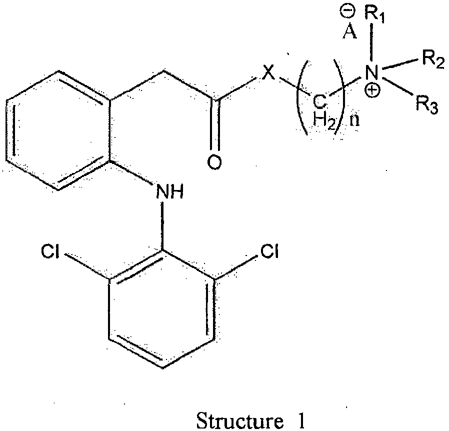

In structure 1, R₁ represents H, one of any alkyl, alkyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, X represents O, or S; A⁻ represents any negative ions; and n=0,1,2,3,4,5,6,7,8,9,10.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

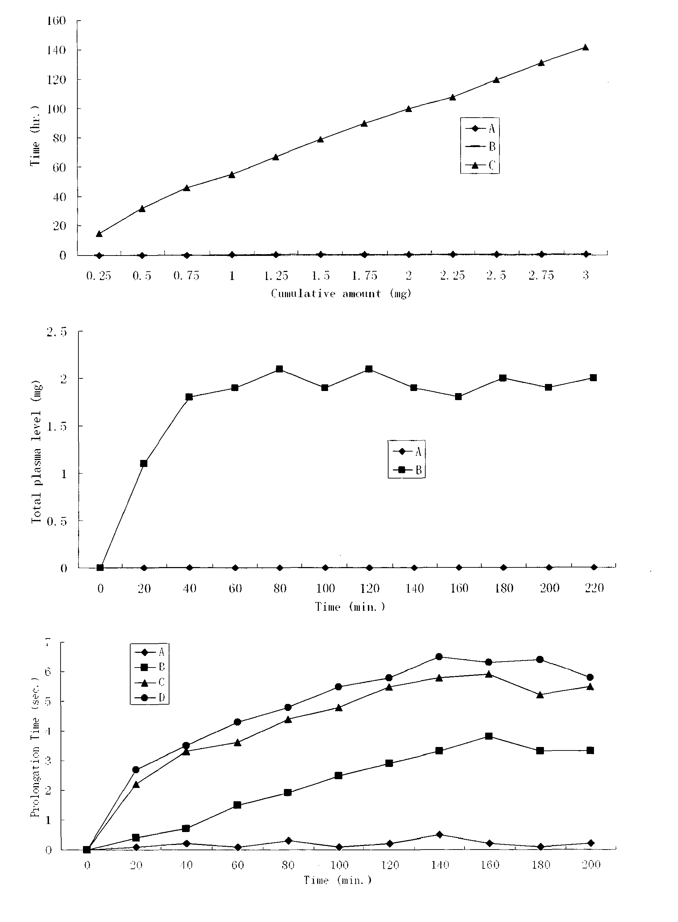

The goal of this invention is to avoid the side effects of diclofenac by increasing the solubility of diclofenac in gastric juice and the penetration rate of diclofenac through the membrane and skin barrier which will make it administrable transdermally (topical application). These novel pro-drugs of diclofenac have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of diethylaminoethyl 2[(2,6-dichlorophenyl)amino]benzene acetate.AcOH and 2[(2,6-dichlorophenyl)amino]benzene acetic acid (diclofenac) in water were >400 mg and 0.1 mg/ml. In many instances, the slowest or rate-limiting step in the sequence is the dissolution of the drug. Diclofenac has a very low solubility in gastric juice. It stays in the GI tract for a long time and thus, may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part of the pro-drugs will push the pro-drug into the cytosol, a semi-liquid concentrated aqueous solution or suspension. Due to the short stay in GI tract, the pro-drugs will not cause gastric mucosal cell damage. The penetration rates of diethylaminoethyl 2[(2,6-dichlorophenyl)amino]benzene acetate.AcOH and 2[(2,6-dichlorophenyl)amino]benzene acetic acid (diclofenac) through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of diethylaminoethyl 2[(2,6-dichlorophenyl)amino]benzene acetate.AcOH and 2[(2,6-dichlorophenyl)amino ]benzene acetic acid (diclofenac) penetrating the skin versus time were determined by a specific high-performance liquid chromatography method. The results using a donor consisting of either a 30% suspension of 2[(2,6-dichlorophenyl)amino]benzene acetic acid (diclofenac) and ethyl 2[(2,6-dichlorophenyl)amino]benzene acetate and 30% solution of diethylaminoethyl 2[(2,6-dichlorophenyl)amino]benzene acetate.AcOH in 2mL of pH 7.4 phosphate buffer (0.2M) are shown in Figure 1. Apparent flux values of 0.2 mg, 0.2 mg and 50 mg/cm²/h were calculated for 2[(2,6-dichlorophenyl)amino] benzene acetic acid (diclofenac), ethyl 2[(2,6-dichlorophenyl)amino]benzene acetate (the non positive charged normal ester of diclofenac) and diethylaminoethyl 2[(2,6-dichlorophenyl)amino]benzene acetate.AcOH. The results suggest that the pro-drug, diethylaminoethyl 2[(2,6-dichlorophenyl)amino]benzene acetate.AcOH diffuses through human skin ∼250 times faster than do 2[(2,6-dichlorophenyl)amino]benzene acetic acid (diclofenac) and ethyl 2[(2,6-dichlorophenyl)amino]benzene acetate. The normal ester, ethyl 2[(2,6-dichlorophenyl)amino]benzene acetate and diclofenac itself have same penetration rate. The results suggest that the positive charge on the di-alkyaminoethyl group has a very important role in the passage of the drug across the membrane and skin barrier. Other prodrugs of 'Structure 1' have very high penetration rates and are very close to that of diethylaminoethyl 2[(2,6-dichlorophenyl)amino]benzene acetate.AcOH.

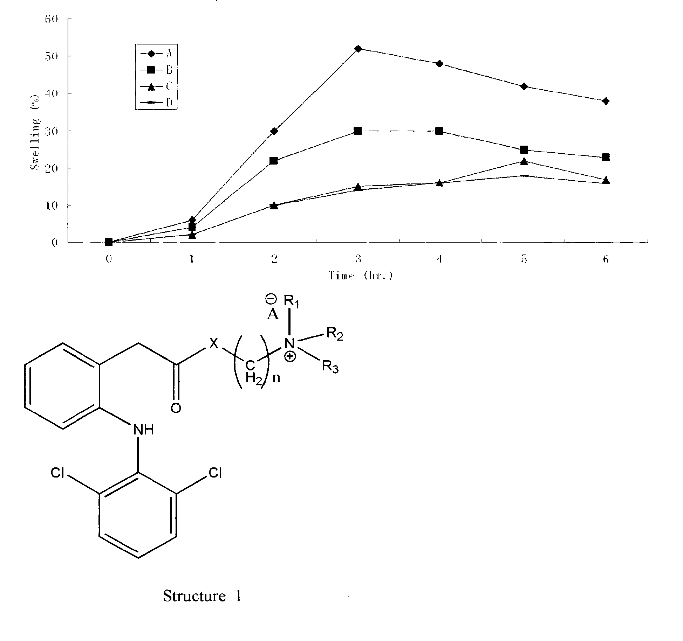

The in vivo rates of penetration of diethylaminoethyl 2[(2,6-dichlorophenyl)amino] benzene acetate.AcOH and 2[(2,6-dichlorophenyl)amino]benzene acetic acid (diclofenac) through the skin of intact hairless mice were compared. The donor consisted of a 20% solution of diethylaminoethyl 2[(2,6-dichlorophenyl)amino] benzene acetate.AcOH and 2[(2,6-dichlorophenyl)amino]benzene acetic acid (diclofenac) in 1 mL of isopropanol applied to a 1 cm² on the backs of the hairless mice. The results (Figure 2) show that the peak levels of diclofenac was reached ∼40 minutes after application of the donor systems. It takes 1-2 hours for diclofenac to reach the peak diclofenac level when it is taken orally. The peaks were ∼0.001 mg/ml for diclofenac or ∼2.1 mg/ml for diethylaminoethyl 2[(2,6-dichlorophenyl)amino] benzene acetate.AcOH (approximately 2100 times difference). ∼2.1 mg/ml of diclofenac in plasma is more than 1000 times higher than the diclofenac plasma level for effective analgesia and effective anti-inflammatory activity (-0.002 mg/ml). This is a very exciting result. It will be very easy and fast to deliver therapeutically effective plasma levels of diclofenac into the host by these pro-drugs. These results suggest that the pro-drugs can be administered not only orally, but also transdermally for any kind of medical treatments. The in vivo rates of penetration of other pro-drugs of 'Structure 1' are close to that of diethylaminoethyl 2[(2,6-dichlorophenyl)amino]benzene acetate.AcOH.

To check the gastroduodenal bleeding caused by drugs, rats (two groups, each group had 10 rats) were orally administered with 25 mg/kg of diclofenac or diethylaminoethyl 2[(2,6-dichlorophenyl)amino]benzene acetate.AcOH per day for 21 days. We found an average of 3 mg of fecal blood per gram of feces in the diclofenac group and none in diethylaminoethyl 2[(2,6-dichlorophenyl)amino]benzene acetate.AcOH group.

The acute toxicity of the prodrugs was investigated. The LD₅₀ orally in rats are: 0.75 g/kg and 0.7 g for diethylaminoethyl 2[(2,6-dichlorophenyl)amino]benzene acetate.AcOH and dimethylaminoethyl 2[(2,6-dichlorophenyl)amino]benzene acetate.AcOH. The results show that the prodrugs are less toxic than diclofenac (LD₅₀ =0.45g/kg).

Diclofenac has demonstrated anti-inflammatory, analgesic, antipyretic, and antirheumatic activity. A good prodrug should go back to the drug itself in plasma. Diethylaminoethyl ester group of diethylaminoethyl 2[(2,6-dichlorophenyl)amino] benzene acetate.AcOH can be rapidly cleaved by the enzymes in human plasma in vitro and more than 90% of the pro-drug is changed back to diclofenac. Due to the pro-drug having a much better absorption rate, the prodrug will have more strength than the diclofenac itself at the same dosage. The analgetic, antipyretic, and anti-inflammatory activities of diethylaminoethyl 2[(2,6-dichlorophenyl)amino]benzene acetate.AcOH were tested using diclofenac as a comparison. Other compounds of the general 'Structure 1' were tested by the same methods and have very similar results as that of diethylaminoethyl 2[(2,6-dichlorophenyl)amino]benzene acetate.AcOH.

Analgetic activity: The prolongation time of pain the threshold of a mouse tail was determined in accordance with the D'Amour-Smith Method (J. Pharmacol. Exp. Ther., 72, 74(1941)). After25 mg/kg of diclofenac was administered orally and 25 mg/kg of diethylaminoethyl 2[(2,6-dichlorophenyl)amino]benzene acetate.AcOH was administered orally and transdermally, the tails of mice were exposed to heat and the prolongation time of pain threshold was determined. The results obtained are shown in Figure 3. The groups administered 25 mg/kg of diethylaminoethyl 2[(2,6-dichlorophenyl)amino]benzene acetate.AcOH orally (C) and transdermally (D) were shown to exhibit stronger analgetic activity than the group administered 25 mg/kg of diclofenac.

The number of writhings that occurred when mice were administered an acetic acid solution intraperitoneally were counted, and the rate of inhibition based on the control group was calculated. 42 mice were divided into 7 groups (6 mice each). Diclofenac (10 mg and 20 mg/kg) was administered to groups B1 and B2 of mice and diethylaminoethyl 2[(2,6-dichlorophenyl)amino]benzene acetate.AcOH (10 mg and 20 mg/ kg) was administered orally to groups C1 and C2. Diethylaminoethyl 2[(2,6-dichlorophenyl)amino]benzene acetate.AcOH (10 mg and 20 mg/kg) was administered transdermally to groups D1 and D2. The A group is the control group. The test compounds were administered to the mice 30 minutes before the acetic acid solution was administered. The results are shown in Table 1.

**Table 1. The rate of writhings inhibition by diclofenac and its pro-drugs**

| Group | A | B1 | B2 | C1 | C2 | D1 | D2 |
|---|---|---|---|---|---|---|---|
| Dose (mg/kg) | 0 | 10 | 20 | 10 | 20 | 10 | 20 |
| No. of writhings | 34.2 | 14.2 | 10.1 | 12.1 | 9.2 | 10.3 | 8.8 |
| % | - | 58.5 | 70.5 | 64.6 | 73.1 | 69.9 | 74.3 |

The results show that diethylaminoethyl 2[(2,6-dichlorophenyl)amino]benzene acetate.AcOH demonstrates better analgetic activity than diclofenac. Other compounds of 'Structure 1' show similar analgetic activity.

Antipyretic activity: Rats received a sterilized E. coli suspension as a pyrogen. 56 rats were divided into 7 groups. The control group is group A. 2 hours later, diclofenac (B1 for 10 mg/kg and B2 for 20 mg/kg) was administered orally and diethylaminoethyl 2[(2,6-dichlorophenyl)amino]benzene acetate.AcOH was administered orally (C1 for 10 mg/kg and C2 for 20 mg/kg) and transdermally (D1 for 10 mg/kg and D2 for 20 mg/kg). The body temperatures of rats were taken at 90 min. intervals before and after the administration of the test compounds. The results are shown in Table 2.

**Table 2. Antipyretic activity of diclofenac and its pro-drugs**

| Compound | t=0 min. | t=90 min. | t=180 min. | t=270 min. |
|---|---|---|---|---|
| Control group(A) | 37.56±0.05 | 37.55±0.07 | 37.53±0.05 | 37.52±0.08 |
| (10mg/kg, B1) | 37.56±0.06 | 36.90±0.05 | 36.91±0.08 | 36.92±0.07 |
| (20mg/kg, B2) | 37.55±0.09 | 36.60±0.07 | 36.53±0.06 | 36.55±0.05 |
| (10mg/kg, C1, orally) | 37.52±0.07 | 36.50±0.06 | 36.60±0.05 | 36.55±0.08 |
| (20mg/kg, C2, orally) | 37.54±0.08 | 36.30±0.05 | 36.35±0.07 | 36.38±0.08 |
| (10mg/kg, D1, transdermally) | 37.58±0.06 | 36.30±0.06 | 36.35±0.08 | 36.311±0.07 |
| (20mg/kg, D2, transdermally) | 37.59±0.05 | 36.25±0.05 | 36.30±0.07 | 36.20±0.05 |

The results shown that diethylaminoethyl 2[(2,6-dichlorophenyl)amino]benzene acetate.AcOH demonstrated antipyretic activity at 10 mg/kg and 20 mg/kg dose and better than that of diclofenac. The results show that transdermal administration of diethylaminoethyl 2[(2,6-dichlorophenyl)amino]benzene acetate.AcOH is better than oral administration. Other compounds of 'Structure 1' show similar antipyretic activity.

Anti-inflammatory activity: 10 mg/kg of diethylaminoethyl 2[(2,6-dichlorophenyl)amino]benzene acetate.AcOH was administered orally or transdermally to rats and 10 mg/kg of diclofenac was administered orally. 60 minutes later, a carrageenin solution was administered subcutaneously to the foot pads of the rats. The volume of the hind paw was measured at every hour after the administration of the carrageenin, and the rate of increase in the volume of the paw was calculated and designated as the rate of swelling (%). The results obtained are shown in Figure 4. The results show that diethylaminoethyl 2[(2,6-dichlorophenyl)amino] benzene acetate.AcOH by oral administration and transdermal administration demonstrated better Anti-inflammatory activity than that of diclofenac at 10 mg/kg by oral administration. Other compounds of 'Structure 1' show similar anti-inflammatory activity.

The compounds of formula (1) 'Structure 1' indicated above can be prepared from diclofenac or from functional derivatives of diclofenac. For example, acid halides or mixed anhydrides of formula (2) 'Structure 2'. 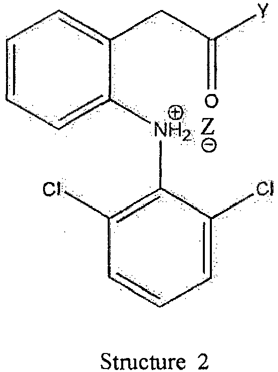 In structure 2, Y represents halogen, alkoxycarbonyl or substituted aryloxy-carbonyloxy, Z represents halogen or other negative ions, by reaction with compounds of formula (3) 'Structure 3' , 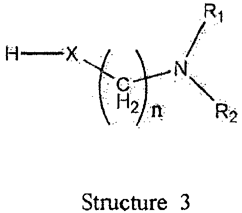 In structure 3, R₁ represents H, one of any alkyl, alkyloxy, alkenyl, or alkynyl residues having 1 to 12 carbon atoms, or aryl residues; R₂ represents H, one of any alkyl, alkyloxy, alkenyl or alkynyl residues having 1 to 12 carbon atoms, or aryl residues; X represents O, S or NH; and n=0,1,2,3,4,5,6,7,8,9,10.

The compounds of formula (1) 'Structure 1' indicated above can be prepared from diclofenac, by reaction with compounds of formula (3) 'Structure 3' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate.

When X represents O, the compounds of formula (1) 'Structure 1' indicated above can be prepared from metal salts or organic base salts of diclofenac, by reaction with compounds of formula (4) 'Structure 4'. 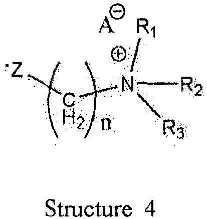 In structure 4, R₁ represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R₃ represents H; Z represents halogen, or p-toluenesulphonyl, A⁻ represents any negative ions; and n=0,1,2,3,4,5,6,7,8,9,10.

When X represents O, the compounds of formula (1) 'Structure 1' indicated above can be prepared from immobilized base salts of diclofenac of formula (5) 'Structure 5', 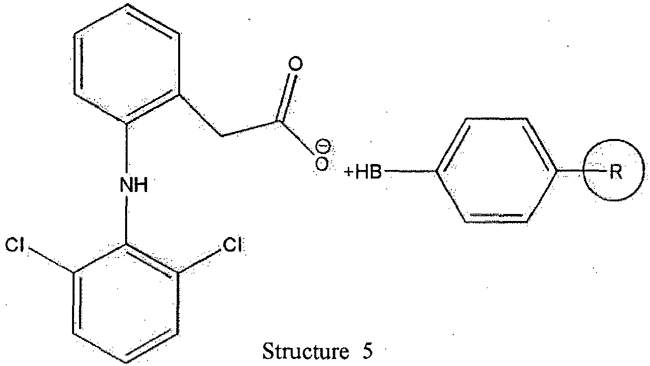 In structure 5, R represents cross-linked resin; B represents any base groups, such as pyridine, piperidine, triethylamine, or other base groups, by reaction with compounds of formula (4) 'Structure 4'.

The present invention relates to pharmaceutical preparations comprising of prodrugs of diclofenac of 'Structure 1' in addition to customary auxiliaries and excipients, in the form of tablets, capsules or solutions for administration orally and in the form of solutions, lotion, ointment, emulsion or gel for administration transdermally. The new active compounds of 'Structure 1' can be combined with vitamins such as A, B, C or E or beta-carotene, or other pharmaceuticals, such as folic acid, etc. for treating any diclofenac-treatable conditions in humans or animals.

It is also known that diclofenac shows an anti-reactive-antiasthmatic activity by inhibition of the cyclooxygenase activity. Due to their very high membrane penetration rate, these pro-drugs can be used in treating asthma by spraying into the mouth or nose of the host.

They can also be used to treat acne and other skin disorders due to their anti-inflammatory properties. They can be used for the treatment and prevention of endothelia dysfunction as well.

These pro-drugs are water-soluble neutral salt and can be tolerated very well by the eye. They can be used for treating eye inflammatory diseases, for treating of ocular pain after corneal surgery, for treating glaucoma or for treating ear inflammatory and/ or painful conditions (otitis).

Transdermal therapeutic application systems of compounds of 'Structure 1' or a composition comprising of at least one compound of 'Structure 1,' as an active ingredient, can be used for treating any diclofenac-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables diclofenac to reach constantly optimal therapeutic blood levels to increase effectiveness and reduce the side effects of diclofenac. These systems can be worn on the wrist, ankle, arm, leg, or any part of body.

### Advantageous Effects

These pro-drugs of diclofenac have a lipophilic portion and a hydrophilic portion (the amine groups that exist in the protonated form at physiological pH). The positively charged amino groups of these pro-drugs have two major advantages. First, it largely increases the solubility of the drugs in water; when these new pro-drugs are administered orally in a dosage form such as a tablet, capsule, solution, or suspension, they will dissolve in gastric juice immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay in the GI tract, the pro-drugs will not cause gastric mucosal cell damage. Experiment results show that more than 90% of the pro-drug was changed back to the drug itself. The pro-drugs have a much better absorption rate, and thus the pro-drugs will have better strength than the diclofenac itself at the same dosage. The experiment results suggest that the pro-drug, diethylaminoethyl 2[(2,6-dichlorophenyl)amino]benzene acetate.AcOH, diffuses through human skin ∼250 times faster than diclofenac itself and ethyl 2[(2,6-dichlorophenyl)amino]benzene acetate. The in vivo rates of penetration of diethylaminoethyl 2[(2,6-dichlorophenyl)amino]benzene acetate.AcOH through the skin of intact hairless mice were very high. It takes 1-2 hours for diclofenac tablets to reach the peak diclofenac plasma level when it is taken orally, but diethylaminoethyl 2[(2,6-dichlorophenyl)amino]benzene acetate.AcOH only took about 30 minutes to reach the peak diclofenac plasma level. The most exciting result is that the pro-drugs can be administered not only orally, but also transdermally for any type of medical treatment and should avoid most of the side effects of diclofenac, most notably GI disturbances such as dyspepsia, gastroduodenal bleeding, gastric ulcerations, and gastritis. Another great benefit of transdermal administration of these pro-drugs is that administering medication, especially to children, will be much easier.

### Description of Drawings

Figure 1: Cumulative amounts of 2[(2,6-dichlorophenyl)amino]benzene acetic acid (diclofenac, A), ethyl 2[(2,6-dichlorophenyl)amino]benzene acetate (the non positive charged normal ester of diclofenac, B) and diethylaminoethyl 2[(2,6-dichlorophenyl)amino]benzene acetate.AcOH (C) crossing isolated human skin tissue in Franz cells (n=5). Diclofenac and ethyl 2[(2,6-dichlorophenyl)amino]benzene acetate were applied as a 30% suspension; diethylaminoethyl 2[(2,6-dichlorophenyl)amino]benzene acetate.AcOH was applied as 30% solution. In each case, the vehicle was pH 7.4 phosphate buffer (0.2 M).

Figure 2: Total plasma levels of diclofenac after topical application of 1 ml of a 20% solution of diethylaminoethyl 2[(2,6-dichlorophenyl)amino]benzene acetate.AcOH and 2[(2,6-dichlorophenyl)amino]benzene acetic acid (diclofenac) in isopropanol to the backs of hairless mice (n=5).

Figure 3: The prolongation time of the pain threshold of mice tails after 25 mg/kg of diclofenac (B) was administered orally, 25 mg/kg of diethylaminoethyl 2[(2,6-dichlorophenyl)amino]benzene acetate.AcOH was administered orally (C) and transdermally (D). A is the control line.

Figure 4. The rate of swelling (%) after a carrageenin injection. 1 hour before the carrageenin injection, 10 mg/kg of diclofenac was administered orally (B), 10 mg/kg of diethylaminoethyl 2[(2,6-dichlorophenyl)amino]benzene acetate.AcOH was administered orally (C) and transdermally (D). A group is the control group.

Structure 1: In structure 1, R₁ represents H, one of any alkyl, alkyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H; X represents O, or S; A⁻ represents any negative ions; and n=0,1,2,3,4,5,6,7,8,9,10.

### Best Mode

### Preparation of diethylaminoethyl 2[(2,6-dichlorophenyl)amino]benzene acetate.AcOH

35.1 g (0.1 mol) of 2[(2,6-dichlorophenyl)amino]benzene acetyl chloride hydrochloride was dissolved in 100 ml of chloroform. The mixture was cooled to 0°C. 30 ml of triethylamine and 11.7 g of diethylaminoethanol were added into the reaction mixture. The mixture is stirred for 3 hours at RT. The solid side product was removed by filtration and washed with chloroform (3 x 30 ml). 6 g of acetic acid is added into the reaction mixture with stirring. The organic solution was evaporated off. After drying, it yielded 39 g of the desired product (85.6 %). Hygroscopic product; Solubility in water: 400 mg/ml; Elementary analysis: C₂₂H₂₈Cl₂N₂O₄; MW: 455.37. Calculated % C: 58.03; H: 6.20; Cl: 15.57; N: 6.15; O: 14.05; Found % C: 58.01; H: 6.22; Cl: 15.55, N: 6.14; O: 14.09. ¹H-NMR (400 MHz, CDCl₃): δ: 1.56 (t, 6H), 2.21 (s, 3H), 3.28 (m, 4H), 3.50 (s, 2 H), 3.52 (m, 2H), 3.81 (b, 1H), 4.51 (t, 2H), 6.32 (d, 1H), 6.50 (m, 2 H), 6.78 (b, 1H), 6.82 (m, 2H), 6.91 (d, 2H).

### Mode for Invention

### Preparation of dimethylaminoethyl 2[(2,6-dichlorophenyl)amino] benzene acetate.AcOH

2[(2,6-Dichlorophenyl)amino]benzene acetyl chloride hydrochloride (35.1 g, 0.1 mol) was dissolved in 100 ml of acetone. The mixture was cooled to 0°C. Dimethylaminoethanol (8.9 g, 0.1 mol) were added into the reaction mixture. Sodium bicarbonate (20 g) and water (100 ml) are added into the mixture. The mixture is stirred for 3 hours at RT. The solution is evaporated to dryness. Acetone (100 ml) is added into the residue. The solid side product was removed by filtration and washed with acetone (3 x 30 ml). 6 g of acetic acid is added into the reaction mixture with stirring. The organic solution was evaporated off. After drying, it yielded 38 g of the desired product (88.9 %). Hygroscopic product; Solubility in water: 410 mg/ml; Elementary analysis: C₂₀H₂₄Cl₂N₂O₄; MW: 427.32. Calculated % C: 56.21; H: 5.66; Cl: 16.59, N: 6.56; O: 14.98; Found % C: 56.18; H: 5.68; Cl: 16.56, N: 6.55; O: 15.03. ¹H-NMR (400 MHz, CDCl₃): δ: 2.21 (s, 3H), 2.91 (s, 6H), 3.50 (s, 2 H), 3.52 (m, 2H), 3.81 (b, 1H), 4.51 (t, 2H), 6.32 (d, 1H), 6.50 (m, 2 H), 6.78 (b, 1H), 6.82 (m, 2H), 6.91 (d, 2H).

### Preparation of S-dimethylaminoethyl 2[(2,6-dichlorophenyl)amino] benzene acetate.AcOH

2[(2,6-Dichlorophenyl)amino]benzene acetyl chloride hydrochloride (35.1 g, 0.1 mol) was dissolved in 100 ml of acetone. The mixture was cooled to 0°C. Dimethylaminoethyl mercaptan (9.3 g, 0.1 mol) were added into the reaction mixture. Sodium bicarbonate (20 g) and water (100 ml) are added into the mixture. The mixture is stirred for 3 hours at RT. The solution is evaporated to dryness. Acetone (100 ml) is added into the residue. The solid side product was removed by filtration and washed with acetone (3 x 30 ml). 6 g of acetic acid is added into the reaction mixture with stirring. The organic solution was evaporated off. After drying, it yielded 40 g of the desired product (90.2 %). Hygroscopic product; Solubility in water: 410 mg/ml; Elementary analysis: C₂₀H₂₄Cl₂N₂O₃S MW: 443.39. Calculated % C: 54.18; H: 5.46; Cl: 15.99, N: 6.32; O: 10.83, S: 7.22; Found % C: 54.16; H: 5.48; Cl: 15.97, N: 6.31; O: 10.86, S: 7.23. ¹H-NMR (400 MHz, CDCl₃): δ: 2.21 (s, 3H), 2.91 (s, 6H), 3.31 (t, 2H), 3.66 (s, 2 H), 3.91 (m, 2H), 3.93 (b, 1H), 6.32 (d, 1H), 6.50 (m, 2 H), 6.78 (b, 1H), 6.82 (m, 2H), 6.91 (d, 2H).

### Preparation of N-dimethylaminoethyl 2[(2,6-dichlorophenyl)amino] benzene acetamide.AcOH

2[(2,6-Dichlorophenyl)amino]benzene acetyl chloride hydrochloride (35.1 g, 0.1 mol) was dissolved in 100 ml of acetone. The mixture was cooled to 0°C. Dimethylaminoethylamine (8.9 g) added into the reaction mixture. Sodium bicarbonate (20 g) and water (100 ml) are added into the mixture. The mixture is stirred for 3 hours at RT. The solution is evaporated to dryness. Acetone (100 ml) is added into the residue. The solid side product was removed by filtration and washed with acetone (3 x 30 ml). 6 g of acetic acid is added into the reaction mixture with stirring. The organic solution was evaporated off. After drying, it yielded 40 g of the desired product (93.8 %). Hygroscopic product; Solubility in water: 450 mg/ml; Elementary analysis: C₂₀H₂₅Cl₂N₂O ₃; MW: 426.34. Calculated % C: 56.34; H: 5.91; Cl: 16.63, N: 9.86; O: 11.26; Found % C: 56.31; H: 5.5.94; Cl: 16.61, N: 9.84; O: 11.30. ¹H-NMR (400 MHz, CDCl₃): δ: 2.21 (s, 3H), 2.91 (s, 6H), 3.44 (s, 2 H), 3.51 (t, 2H), 3.64 (t, 2H), 3.93 (b, 1H), 6.32 (d, 1H), 6.50 (m, 2 H), 6.78 (b, 1H), 6.82 (m, 2H), 6.91 (d, 2H), 8.0 (b, 1H).

### Preparation of dipropylaminoethyl 2[(2,6-dichlorophenyl)amino] benzene acetate.AcOH

31.8 g (0.1 mol) of sodium 2[(2,6-dichlorophenyl)amino] benzene acetate was suspended in 180 ml of chloroform. 28.8 g (0.1 mol) of dipropylaminoethyl bromide.HBr was added into the mixture and the mixture was stirred for 5 hours at RT. 8.2 g (0.1 mol) of sodium acetate was added into the reaction mixture with stirring. The mixture is stirred for 2 hours. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 41 g of the desired product (87%). Hygroscopic product; Solubility in water: 300 mg/ml; Elementary analysis: C₂₄H₃₂Cl₂N₂O₄; MW:483.43 Calculated % C: 59.63; H: 6.67; Cl: 14.67; N: 5.79; O: 13.24; Found % C: 59.60; H: 6.70; Cl: 14.65, N: 5.78; O: 13.27. ¹H-NMR (400 MHz, CDCl₃): δ: 0.97 (t, 6H), 1.78 (m, 4H), 2.21 (s, 3H), 3.24 (t, 4H), 3.50 (s, 2 H), 3.52 (m, 2H), 3.81 (b, 1H), 4.51 (t, 2H), 6.34 (d, 1H), 6.50 (m, 2 H), 6.78 (b, 1H), 6.82 (m, 2H), 6.91 (d, 2H).

### Preparation of dipropylaminoethyl dipropylaminoethyl 2[(2,6-dichlorophenyl)amino] benzene acetate.AcOH

60 g of Polymer-bound triethylamine (3 mmol/g, 100-200 mesh) was suspended in 180 ml of chloroform. 31.8 g (0.1 mol) of 2[(2,6-dichlorophenyl)amino] benzene acetic acid was added into the mixture with stirring. 43 g (0.15ml) of dipropylaminoethyl bromide.HBr was added into the mixture and the mixture was stirred for 5 hours at RT. The polymer is removed by filtration and washed with tetrahydrofuran (3 x 50 ml). 8.2 g (0.1 mol) of sodium acetate was added into the reaction mixture with stirring. The mixture is stirred for 2 h. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 45 g of the desired product (93.2%). Hygroscopic product; Solubility in water: 300 mg/ml; Elem entary analysis: C₂₄H₃₂Cl₂N₂O₄; MW: 483.43 Calculated % C: 59.63; H: 6.67; Cl: 14.67; N: 5.79; O: 13.24; Found % C: 59.60; H: 6.70; Cl: 14.65, N: 5.78; O: 13.27. ¹H-NMR (400 MHz, CDCl₃): δ: 0.97 (t, 6H), 1.78 (m, 4H), 2.21 (s, 3H), 3.24 (t, 4H), 3.50 (s, 2 H), 3.52 (m, 2H), 3.81 (b, 1H), 4.51 (t, 2H), 6.34 (d, 1H), 6.50 (m, 2 H), 6.78 (b, 1H), 6.82 (m, 2H), 6.91 (d, 2H).

### Industrial Applicability

The pro-drugs of formula (1) 'Structure 1' are superior to diclofenac. They may be used medicinally in treating any diclofenac-treatable conditions in humans or animals. They can be used for the relief of signs and symptoms of rheumatoid arthritis, osteoarthritis and ankylosing spondylitis, for the treatment of dysmenorrhea. They can be used alone or as an adjunct in the treatment of biliary colic, fever, and episiotomy pain. They are also used in treatment of gout, acute migraine headaches, and renal colic and in the treatment of postoperative inflammation in patients who have undergone cataract extraction. They may be used in the prevention of cancer. Due to their very high membrane penetration rate, these pro-drugs can be used in treating asthma by inhalation to a host. They can be used to treat acne due to their anti-inflammatory properties. These pro-drugs are water-soluble neutral salt and can be tolerated very well by the eye. They can be used for treating eye inflammatory diseases, for treating of ocular pain after corneal surgery, for treating glaucoma or for treating ear inflammatory and/or painful conditions (otitis).

## Claims

1. The compounds of formula (1) 'Structure 1', 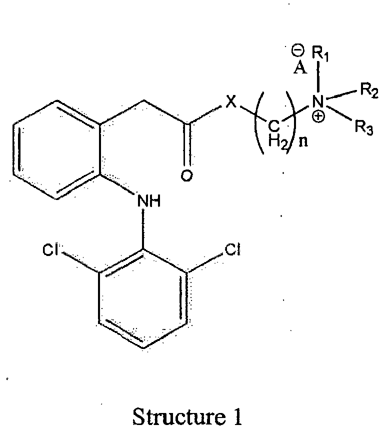 wherein
R₁ represents H, one of any alkyl, alkyloxy, alkenyl, or alkynyl residues having 1 to 12 carbon atoms, or aryl residues,
R₂ represents H, one of any alkyl, alkyloxy, alkenyl or alkynyl residues having 1 to 12 carbon atoms, or aryl residues,
R₃ represents H,
X represents O or S,
A- represents any negative ion, and
n=0,1,2,3,4,5,6,7,8,9,10.

2. A process for the preparation of compounds of formula (1) 'Structure 1' according to Claim 1, wherein
a) acid halides or mixed anhydrides of formula (2) 'Structure 2' 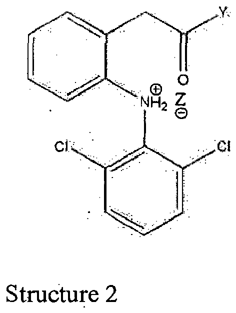 are reacted with compounds of formula (3) 'Structure 3', 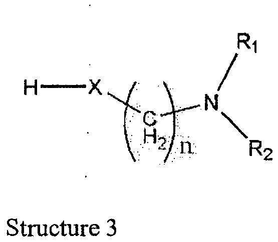 wherein in Structure 2
Y represents halogen, alkoxycarbonyl or substituted aryloxy-carbonyloxy,
Z represents halogen or other negative ions,
and in Structure 3,
R₁ represents H, one of any alkyl, alkyloxy, alkenyl, or alkynyl residues having 1 to 12 carbon atoms, or aryl residues,
R₂ represents H, one of any alkyl, alkyloxy, alkenyl or alkynyl residues having 1 to 12 carbon atoms, or aryl residues,
X represents O or S, and
n=0, 1, 2, 3, 4, 5, 6, 7, 8, 9, 10;
b) a metal salt or organic base salt of diclofenac is reacted with compounds of formula (4) 'Structure 4', 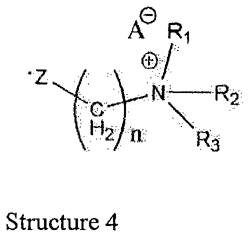 wherein in Structure 4
R₁ represents H, one of any alkyl, alkyloxy, alkenyl, or alkynyl residues having 1 to 12 carbon atoms, or aryl residues,
R₂ represents H, one of any alkyl, alkyloxy, alkenyl, or alkynyl residues having 1 to 12 carbon atoms, or aryl residues,
R₃ represents H,
Z represents halogen, or p-toluenesulphonyl,
A⁻ represents any negative ion, and
n=0,1, 2, 3, 4, 5, 6, 7, 8, 9, 10.

3. The process for the preparation of compounds of formula (1) 'Structure 1' according to claim 2(a), wherein
diclofenac is reacted with compounds of formula (3) 'Structure 3' 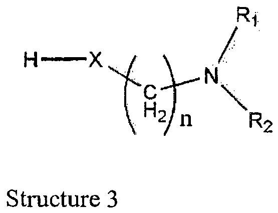 by using coupling reagents, such as N,N'-Dicyclohexylcarbodiimide, N, N'-Diisopropylcarbodiimide, O-(Benzotriazol-1 -yl)-N,N,N',N'-tetramethyluronium tetrafluoroborate, O-(Benzotriazol-1 -yl)-N,N,N',N'-tetramethyluronium hexafluorophosphate, Benzotriazol-1-yl-oxy-tris (dimethylamino)phosphonium hexafluorophosphate,
wherein in Structure 3
R₁ represents H, one of any alkyl, alkyloxy, alkenyl, or alkynyl residues having 1 to 12 carbon atoms, or aryl residues,
R₂ represents H, one of any alkyl, alkyloxy, alkenyl or alkynyl residues having 1 to 12 carbon atoms, or aryl residues,
X represents O or S,
and n=0, 1, 2, 3, 4, 5, 6, 7, 8, 9, 10.

4. The process for the preparation of compounds of formula (1) 'Structure 1' according to claim 2(b), wherein
an immobilized base salt of diclofenac of formula (5) 'Structure 5' 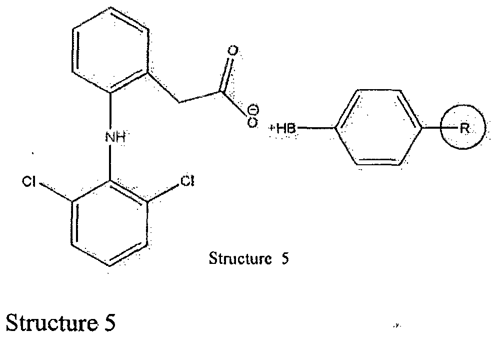 is reacted with compounds of formula (4) 'Structure 4', 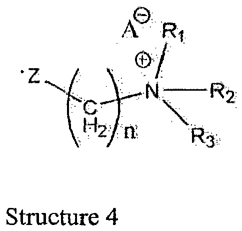 wherein in Structure 4
R₁ represents H, one of any alkyl, alkyloxy, alkenyl, or alkynyl residues having 1 to 12 carbon atoms, or aryl residues,
R₂ represents H, one of any alkyl, alkyloxy, alkenyl, or alkynyl residues having 1 to 12 carbon atoms, or aryl residues,
R₃ represents H,
Z represents halogen, or p-toluenesulphonyl, A⁻ represents any negative ion, and
n=0, 1, 2, 3, 4, 5, 6, 7, 8, 9, 10,
and in Structure 5
R represents cross-linked resin,
B represents any base groups, such as pyridine, piperidine, triethylamine, or other base groups.

5. Compound of claim 1 or a composition comprising at least one compound of claim 1 for use in a method for treating a diclofenac-treatable condition in a human or animal, wherein the diclofenac-treatable condition is pain, an inflammatory disease, cancer, fever or skin disorders, and the compound or composition is administered orally or transdermally.

6. The compound or composition for use according to claim 5, wherein the conditions treated include pain from a toothache, headache, and arthritis and other inflammatory pain, fever, cancer, dysmenorrhea and acute migraine headache.

7. The compound or composition for use according to claims 5 or 6, wherein the compound or composition is administered transdermally to any part of body and in the form of a solution, spray, lotion, ointment, emulsion or gel.

8. The compound or composition for use according to claim 5, wherein the treated condition is headache, toothache, and muscle pain, and arthritis and other inflammatory pain, the compound or composition is topically administered to the inflamed area in a therapeutically effective amount.

9. The compound or composition for use according to claims 5 or 7, wherein the condition treated is acne, sunburn or other skin disorders and wherein the compound or composition is in the form of a solution, spray, lotion, ointment, emulsion or gel and administered transdermally.

10. The compound or composition for use according to claim 5, wherein the condition treated is asthma and the compound or composition is administered by spraying to through the mouth or nose or other parts of body.

11. The compound or composition for use according to claim 5, wherein the condition treated is an eye inflammatory disease.

12. Transdermal therapeutic application systems of a compound of claim 1 or a composition comprising of at least one compound of claim 1 for treating any diclofenac-treatable conditions in humans or animals, wherein the systems are a bandage or a patch comprising of one active substance- containing matrix layer and an impermeable backing layer.

13. The transdermal therapeutic application systems of claim 12, wherein the systems have an active substance reservoir, which has a permeable bottom facing the skin.

## Patentansprüche

1. Eine Verbindung der Formel (1) "Struktur 1" 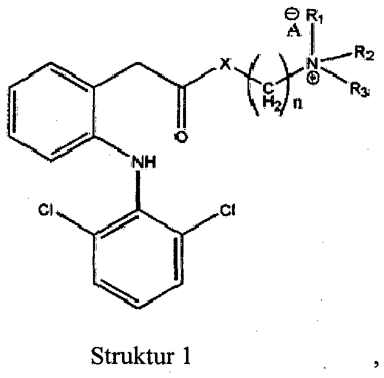 wobei
R₁ H, einen beliebigen Alkyl-, Alkyloxy-, Alkenyl- oder Alkinyl-Rest mit 1 bis 12 Kohlenstoffatomen oder einen Arylrest darstellt,
R₂ H, einen beliebigen Alkyl-, Alkyloxy-, Alkenyl- oder Alkinyl-Rest mit 1 bis 12 Kohlenstoffatomen oder einen Arylrest darstellt,
R₃ H darstellt,
X O oder S darstellt,
A- irgendein negatives Ion darstellt und
n = 0,1,2,3,4,5,6,7,8,9,10 ist.

2. Ein Verfahren zur Herstellung von Verbindungen der Formel (1) "Struktur 1" nach Anspruch 1, wobei
a) Säurehalogenide oder gemischte Anhydride der Formel (2) "Struktur 2" 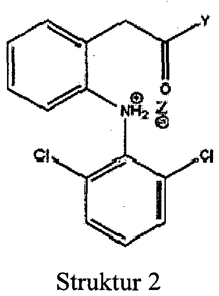 mit Verbindungen der Formel (3) "Struktur 3" umgesetzt werden 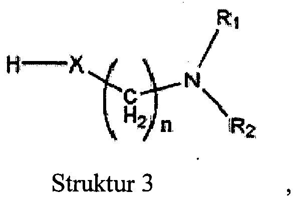 wobei in Struktur 2
Y Halogen, Alkoxycarbonyl oder ein substituiertes Aryloxy-Carbonyloxy darstellt,
Z Halogen oder andere negative Ionen darstellt,
und in Struktur 3,
R₁ H, einen beliebigen Alkyl-, Alkyloxy-, Alkenyl- oder Alkinyl-Rest mit 1 bis 12 Kohlenstoffatomen oder einen Arylrest darstellt,
R₂ H, einen beliebigen Alkyl-, Alkyloxy-, Alkenyl- oder Alkinyl-Rest mit 1 bis 12 Kohlenstoffatomen oder einen Arylrest darstellt,
X O oder S darstellt, und
n = 0, 1, 2, 3, 4, 5, 6, 7, 8, 9, 10 ist;
b) ein Metallsalz oder organisches Basensalz von Diclofenac mit Verbindungen der Formel (4) "Struktur 4" umgesetzt wird 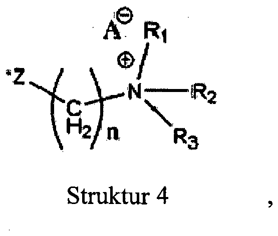 wobei in Struktur 4
R₁ H, einen beliebigen Alkyl-, Alkyloxy-, Alkenyl- oder Alkinyl-Rest mit 1 bis 12 Kohlenstoffatomen oder einen Arylrest darstellt,
R₂ H, einen beliebigen Alkyl-, Alkyloxy-, Alkenyl- oder Alkinyl-Rest mit 1 bis 12 Kohlenstoffatomen oder einen Arylrest darstellt,
R₃ H darstellt,
Z Halogen oder p-Toluolsulfonyl darstellt,
A- irgendein negatives Ion darstellt und
n = 0,1, 2, 3, 4, 5, 6, 7, 8, 9, 10 ist.

3. Das Verfahren zur Herstellung der Verbindungen der Formel (1) "Struktur 1" nach Anspruch 2(a), wobei
Diclofenac mit Verbindungen der Formel (3) "Struktur 3" 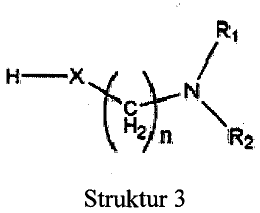 durch Verwendung von Kopplungsreagenzien wie N,N'-Dicyclohexylcarbodiimid, N,N'-Diisopropylcarbodiimid, O-(Benzotriazol-1-yl)-N,N,N',N'-tetramethyluroniumtetrafluorborat, O-(Benzotriazol-1-yl)-N,N,N',N'-tetramethyluroniumhexafluorophosphat, Benzotriazol-1-yl-oxy-tris(dimethylamino)phosphoniumhexafluorophosphat umgesetzt wird,
wobei in Struktur 3
R₁ H, einen beliebigen Alkyl-, Alkyloxy-, Alkenyl- oder Alkinyl-Rest mit 1 bis 12 Kohlenstoffatomen oder einen Arylrest darstellt,
R₂ H, einen beliebigen Alkyl-, Alkyloxy-, Alkenyl- oder Alkinyl-Rest mit 1 bis 12 Kohlenstoffatomen oder einen Arylrest darstellt,
X O oder S darstellt,
und n = 0, 1, 2, 3, 4, 5, 6, 7, 8, 9, 10 ist.

4. Das Verfahren zur Herstellung der Verbindungen der Formel (1) "Struktur 1" nach Anspruch 2(b), wobei
ein immobilisiertes Basensalz von Diclofenac der Formel (5) "Struktur 5" 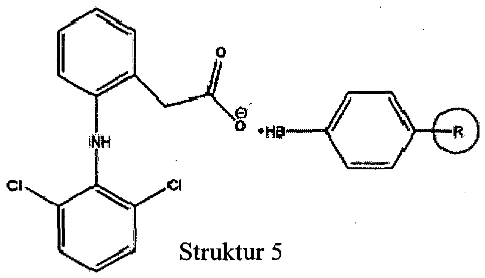 mit Verbindungen der Formel (4) "Struktur 4" umgesetzt wird 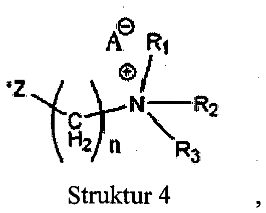 wobei in Struktur 4
R₁ H, einen beliebigen Alkyl-, Alkyloxy-, Alkenyl- oder Alkinyl-Rest mit 1 bis 12 Kohlenstoffatomen oder einen Arylrest darstellt,
R₂ H, einen beliebigen Alkyl-, Alkyloxy-, Alkenyl- oder Alkinyl-Rest mit 1 bis 12 Kohlenstoffatomen oder einen Arylrest darstellt,
R₃ H darstellt,
Z Halogen oder p-Toluolsulfonyl darstellt,
A⁻ irgendein negatives Ion darstellt und
n = 0, 1, 2, 3, 4, 5, 6, 7, 8, 9, 10 ist,
und in Struktur 5
R ein vernetztes Harz darstellt und
B irgendeine Basegruppe, wie Pyridin, Piperidin, Triethylamin oder andere Basegruppen darstellt.

5. Verbindung nach Anspruch 1 oder eine Zusammensetzung, die mindestens eine Verbindung nach Anspruch 1 umfasst, zur Verwendung in einem Verfahren zur Behandlung einer Diclofenac-behandelbaren Krankheit in einem Menschen oder einem Tier, wobei die Diclofenac-behandelbare Krankheit Schmerz, eine Entzündungskrankheit, Krebs, Fieber oder Erkrankungen der Haut ist und die Verbindung oder Zusammensetzung oral oder transdermal verabreicht wird.

6. Die Verbindung oder Zusammensetzung zur Verwendung nach Anspruch 5, wobei die behandelten Zustände Schmerz beinhalten, ausgehend von Zahnschmerz, Kopfschmerz und Arthritis und anderem Entzündungsschmerz, Fieber, Krebs, Dysmenorrhoe und akutem Migränekopfschmerz.

7. Die Verbindung oder Zusammensetzung zur Verwendung nach den Ansprüchen 5 oder 6, wobei die Verbindung oder Zusammensetzung transdermal an jeden Teil des Körpers und in Form einer Lösung, eines Sprays, einer Lotion, einer Salbe, einer Emulsion oder eines Gels verabreicht wird.

8. Die Verbindung oder Zusammensetzung zur Verwendung nach Anspruch 5, wobei der behandelte Zustand Kopfschmerz, Zahnschmerz, Muskelschmerz und Arthritis und ein anderer Entzündungsschmerz ist, wobei die Verbindung oder Zusammensetzung topisch an den entzündeten Bereich in einer therapeutisch wirksamen Menge verabreicht wird.

9. Die Verbindung oder Zusammensetzung zur Verwendung nach den Ansprüchen 5 oder 7, wobei der behandelte Zustand Akne, Sonnenbrand oder andere Hauterkrankungen ist und die Verbindung oder Zusammensetzung in Form einer Lösung, eines Sprays, einer Lotion, einer Salbe, einer Emulsion oder eines Gels ist und transdermal verabreicht wird.

10. Die Verbindung oder Zusammensetzung zur Verwendung nach Anspruch 5, wobei der behandelte Zustand Asthma ist und die Verbindung oder Zusammensetzung durch Sprühen in den Mund oder die Nase oder auf andere Teile des Körpers verabreicht wird.

11. Die Verbindung oder Zusammensetzung zur Verwendung nach Anspruch 5, wobei der behandelte Zustand eine Augenentzündungserkrankung ist.

12. Transdermale therapeutische Anwendungssysteme einer Verbindung nach Anspruch 1 oder einer Zusammensetzung, die mindestens eine Verbindung nach Anspruch 1 umfasst, zur Behandlung von jeder Diclofenac-behandelbaren Krankheit in Menschen oder Tieren, wobei diese Systeme eine Bandage oder ein Pflaster, die/das eine wirkstoffhaltige Matrixschicht, und eine undurchlässige Deckschicht umfasst, sind.

13. Die transdermalen therapeutischen Anwendungssysteme nach Anspruch 12, wobei die Systeme ein Wirkstoffreservoir haben, das einen durchlässigen Boden hat, der der Haut zugewandt ist.

## Revendications

1. Composés de formule 1 correspondant à la structure 1 : 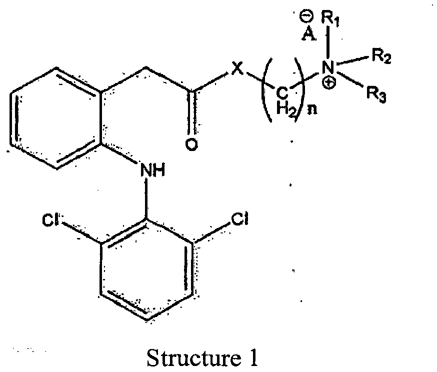 dans laquelle
R₁ représente H ou n'importe quel résidu parmi alkyle, alkyloxy, alcényle ou alcynyle ayant 1 à 12 atomes de carbone, ou aryle,
R₂ représente H ou n'importe quel résidu parmi alkyle, alkyloxy, alcényle ou alcynyle ayant 1 à 12 atomes de carbone, ou aryle,
R₃ représente H,
X représente O ou S,
A⁻ représente n'importe quel ion négatif, et
n vaut 0, 1, 2, 3, 4, 5, 6, 7, 8, 9 ou 10.

2. Procédé pour la préparation de composés de formule 1 correspondant à la structure 1 selon la revendication 1, dans lequel
a) des halogénures d'acide ou des anhydrides mixtes de formule 2 correspondants à la structure 2 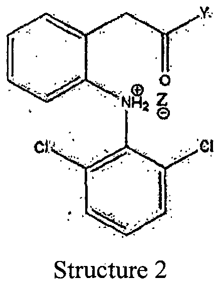 réagissent avec des composés de formule 3 correspondant à la structure 3 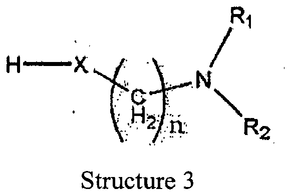 dans laquelle structure 2
Y représente un halogène ou un groupement alcoxycarbonyle ou un groupement substitué aryloxycarbonyloxy,
Z représente un halogène ou d'autres ions négatifs,
et, dans laquelle structure 3,
R₁ représente H ou n'importe quel résidu parmi alkyle, alkyloxy, alcényle ou alcynyle ayant 1 à 12 atomes de carbone, ou aryle,
R₂ représente H ou n'importe quel résidu parmi alkyle, alkyloxy, alcényle ou alcynyle ayant 1 à 12 atomes de carbone, ou aryle,
X représente O ou S, et
n vaut 0, 1, 2, 3, 4, 5, 6, 7, 8, 9 ou 10 ;
b) un sel métallique ou un sel de base organique de diclofénac réagit avec des composés de formule 4 correspondant à la structure 4 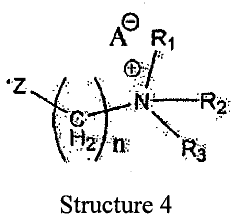 dans laquelle structure 4,
R₁ représente H ou n'importe quel résidu parmi alkyle, alkyloxy, alcényle ou alcynyle ayant 1 à 12 atomes de carbone, ou aryle,
R₂ représente H ou n'importe quel résidu parmi alkyle, alkyloxy, alcényle ou alcynyle ayant 1 à 12 atomes de carbone, ou aryle,
R₃ représente H,
Z représente un halogène ou un groupement p-toluène-sulfonyle,
A⁻ représente n'importe quel ion négatif, et
n vaut 0, 1, 2, 3, 4, 5, 6, 7, 8, 9 ou 10.

3. Procédé pour la préparation de composés de formule 1 correspondant à la structure 1 selon la revendication 2 (a), dans lequel
du diclofénac réagit avec des composés de formule 3 correspondants à la structure 3 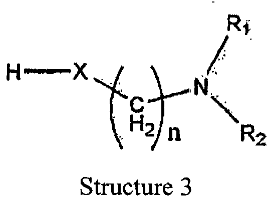 en utilisant des réactifs de couplage, tels que le N,N'-dicyclohexylcarbodiimide, le N,N'-diisopropyl-carbodiimide, le tétrafluoroborate d'O-(benzotriazol-1-yl)-N,N,N',N'-tétraméthyl-uronium, l'hexafluorophosphate d'O-(benzo-triazol-1-yl)-N,N,N',N'-tétraméthyl-uronium, l'hexafluorophosphate de benzotriazol-1-yloxytris(diméthylamino)-phosphonium,
dans laquelle structure 3
R₁ représente H ou n'importe quel résidu parmi alkyle, alkyloxy, alcényle ou alcynyle ayant 1 à 12 atomes de carbone, ou aryle,
R₂ représente H ou n'importe quel résidu parmi alkyle, alkyloxy, alcényle ou alcynyle ayant 1 à 12 atomes de carbone, ou aryle,
X représente O ou S, et
n vaut 0, 1, 2, 3, 4, 5, 6, 7, 8, 9 ou 10.

4. Procédé pour la préparation de composés de formule 1 correspondant à la structure 1 selon la revendication 2 (b), dans lequel
un sel basique immobilisé de diclofénac de formule 5 correspondant à la structure 5 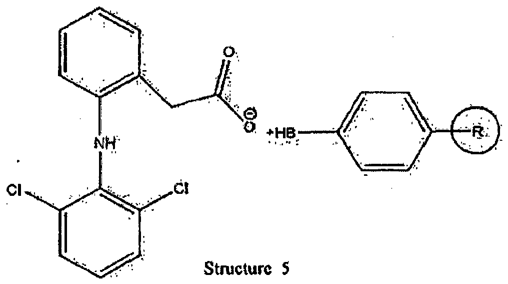 réagit avec des composés de formule 4 correspondant à la structure 4 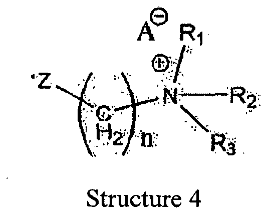 dans laquelle structure 4,
R₁ représente H ou n'importe quel résidu parmi alkyle, alkyloxy, alcényle ou alcynyle ayant 1 à 12 atomes de carbone, ou aryle,
R₂ représente H ou n'importe quel résidu parmi alkyle, alkyloxy, alcényle ou alcynyle ayant 1 à 12 atomes de carbone, ou aryle,
R₃ représente H,
Z représente un halogène ou un groupement p-toluène-sulfonyle, A⁻ représente n'importe quel ion négatif, et
n vaut 0, 1, 2, 3,4, 5, 6, 7, 8, 9 ou 10,
et, dans laquelle structure 5,
R représente une résine réticulée,
B représente n'importe quel groupe basique, tels que pyridine, pipéridine, triéthylamine, ou un autre groupe basique.

5. Composé selon la revendication 1 ou composition comprenant au moins un composé selon la revendication 1 pour utilisation dans un procédé pour le traitement d'un état pouvant être traité avec du diclofénac chez un humain ou un animal, dans lequel (laquelle) l'état pouvant être traité avec du diclofénac est une douleur, une maladie inflammatoire, un cancer, la fièvre ou des troubles cutanés, et le composé ou la composition est administré(e) par voie orale ou transdermique.

6. Composé ou composition pour utilisation selon la revendication 5, dans lequel (laquelle) les états traités comprennent une névralgie dentaire, une céphalée, et une arthrite ainsi que d'autres douleurs inflammatoires, la fièvre, un cancer, une dysménorrhée et une céphalée de type migraine aiguë.

7. Composé ou composition pour utilisation selon la revendication 5 ou 6, lequel composé ou laquelle composition est administré(e) par voie transdermique à n'importe quelle partie du corps et sous la forme d'une solution, d'un spray, d'une lotion, d'une pommade, d'une émulsion ou d'un gel.

8. Composé ou composition pour utilisation selon la revendication 5, dans lequel ou laquelle l'état traité est une céphalée, une névralgie dentaire, une douleur musculaire, ou l'arthrite ou une autre douleur inflammatoire, le composé ou la composition étant administré(e) par voie topique à la zone enflammée en une quantité efficace du point de vue thérapeutique.

9. Composé ou composition pour utilisation selon la revendication 5 ou 7, dans lequel ou laquelle l'état traité est l'acné, un érythème solaire ou un autre trouble cutané, et dans lequel le composé ou dans laquelle la composition est sous la forme d'une solution, d'un spray, d'une lotion, d'une pommade, d'une émulsion ou d'un gel et est administré(e) par voie transdermique.

10. Composé ou composition pour utilisation selon la revendication 5, dans lequel ou laquelle l'état traité est l'asthme, et le composé ou la composition est administré(e) par pulvérisation par la bouche ou le nez ou d'autres parties du corps.

11. Composé ou composition pour utilisation selon la revendication 5, dans lequel ou laquelle l'état traité est une maladie oculaire inflammatoire.

12. Systèmes d'administration thérapeutiques par voie transdermique d'un composé selon la revendication 1 ou d'une composition comprenant au moins un composé selon la revendication 1, pour le traitement de n'importe quel état pouvant être traité avec du diclofénac chez un humain ou un animal, dans lesquels les systèmes sont constitués d'un bandage ou d'une compresse de gaz comprenant une couche de matrice contenant une substance active et une couche d'envers imperméable.

13. Systèmes d'administration thérapeutiques par voie transdermique selon la revendication 12, dans lesquels les systèmes ont un réservoir de substance active, qui a un fond perméable faisant face à la peau.
